# EUROPEAN PATENT SPECIFICATION

(11) **EP 4 331 621 B1**
(45) Date of publication and mention of the grant of the patent: **29.04.2026**
(21) Application number: 23166195.0
(22) Date of filing: 31.03.2023
(51) Int. Cl.: A61K 35/13, A61K 35/17, A61K 49/00, C12N 5/09, G01N 33/50

(54) **MODELS AND METHODS FOR RAPID SCREENING THE EFFICACY OF IMMUNOREGULATING DRUGS**
MODELLE UND VERFAHREN ZUM SCHNELLEN SCREENING DER WIRKSAMKEIT IMMUNREGULIERENDER WIRKSTOFFE
MODELES ET PROCEDES DE CRIBLAGE RAPIDE DE L'EFFICACITE DE MEDICAMENTS IMMUNOREGULATEURS

(30) Priority: 01.09.2022 CN 202211066571
(43) Date of publication of application: 06.03.2024
(73) Proprietor: Shanghai Lide Biotech Co., Ltd., Shanghai 201203 (CN)
(72) Inventor: XIE, Bin, Shanghai, 201203 (CN); WEN, Danyi, Shanghai, 201203 (CN)
(74) Representative: Dehns

(56) References cited:
- WO-A1-2019/196606
- WO-A1-94/25074
- US-A1- 2019 154 663
- DECKER S ET AL: "The hollow fibre model in cancer drug screening - the NCI experience", EUROPEAN JOURNAL OF CANCER, ELSEVIER, 34TH EORTC-NCI-AACR SYMPOSIUM ON MOLECULAR TARGETS AND CANCER THERAPEUTICS, BARCELONA, SPAIN, vol. 40, no. 6, 1 April 2004 (2004-04-01), pages 821 - 826, XP004500008, ISSN: 0959-8049, DOI: 10.1016/J.EJCA.2003.11.029

## Description

### TECHNICAL FIELD

The invention relates to the field of biomedicine, in particular to a model and method for rapid screening the efficacy of immunoregulating drugs and the use of the model.

### BACKGROUND

Currently, a large number of drugs with immune targeting and regulatory functions have been developed and applied to the treatment of numerous clinical diseases. CTLA4 antibody (ipilumumab) and PD1 antibody (Keytruda, Opdivo) are applied in the treatment of tumors such as melanoma, non-small cell lung cancer, small cell lung cancer and head and neck squamous cell carcinoma. TNFα antibody (adalimumab) is used in the treatment of rheumatoid arthritis and active colitis and the like. The latest IL12/IL23p40 antibody (Ustekinumab) is used in the treatment of autoimmune diseases such as psoriasis, inflammatory colitis and systemic lupus erythematosus.

However, not every patient has a positive response to these immunoregulatory drugs. The characteristics of clinical diagnosis and treatment require a lengthy time to test for guidance on treatment regimen. For example, the classic humanized mouse PDX experiment for the modeling for administration of immunological treatments, not only takes 2-5 months, but also fails to meet the diversified needs of clinical patients for the selection of multiple immune drugs. This time also delays the process of new drug development. Therefore, there is a need for rapid and effective functional screening methods that allow precise treatment of patients and identification of biomarkers for different patient populations. Therefore, there is an urgent need for rapid and effective functional screening method that allows precise treatment of patients and identification of biomarkers for different populations.

The current technical solutions disclose drug sensitivity screening of tumor cells in vivo for chemotherapeutic drugs. However, all the cells are tumor cells, and the tested drugs are mainly chemotherapeutic drugs or tumor-targeted drugs (US005676924 A, 1997; US005698413A, 1997; CN108351348 A, 2017), which cannot rapidly screen drugs for immune system targeting or for optimizing drug efficacy.

US 2019/154663 A1 discloses an animal model for screening drugs comprising PVDF capsule tubes (cut-off ca. 500 kDa) implanted into a lab animal. Immune cells are not included in the PVDF capsules and are not exposed to substances used for screening.

WO 94/25074 A1 describes a method of screening chemotherapeutic drugs *in vivo.* It discloses that where fresh tumor tissue is used, cells are dispersed mechanically or enzymatically (using a protein degrading enzyme, such as collagenase) before cultivation. It also discloses that cells are cultured to maintain them in logarithmic growth as appropriate for each cell line; cells are provided with fresh medium 24 hours prior to harvesting in hollow fibers.

WO 2019/196606 A1 discloses a method for screening anti-tumor drugs using a Mini-PDX model. It discloses that tumor tissues, obtained from biopsy/needle biopsy in 1 × collagenase solution, are incubated at 37°C for 1-2 hours. Single cells are collected through a 70 µM strainer, the cell numbers are counted, and the tumor cell concentration is calculated. Cells are then cultivated with conditional reprogramming medium and after tumor cell proliferation, the conditional reprogramming cells are harvested.

DECKERS ET AL ("The hollow fibre model in cancer drug screening - the NCI experience", EUROPEAN JOURNAL OF CANCER, 34TH EORTC-NCI-AACR SYMPOSIUM ON MOLECULAR TARGETS AND CANCER THERAPEUTICS, vol. 40, no. 6, 1 April 2004 (2004-04-01), pages 821-826) disclosed that the *in vivo* hollow fibre model was developed by the National Cancer Institute (NCI) in the United States of America (USA) at a time when the number of potential anti-cancer drugs arising from *in vitro* screening efforts exceeded the available capacity for testing in traditional xenograft models. In the hollow fibre assay, tumour cells are inoculated into hollow (1 mm internal diameter) PVDF fibres, and these are heat-sealed and cut at 2 cm intervals. The fibres are cultured for 24-48 h *in vitro* and then implanted into athymic (nu/nu) mice. DECKERS ET AL does not unambiguously disclose fresh tumor tissue, rather discloses tumor cell lines.

### CONTENT OF THE PRESENT INVENTION

The problem to be solved by the present invention is to provide a model and method for screening the efficacy of immunomodulatory drugs. It overcomes the problems that immune system targeting and optimizing drug efficacy screening are time-consuming and cannot meet the requirements for precise treatment of patients. Compared with traditional immune drug efficacy screening, the present invention can significantly shorten the duration of time for immune drug efficacy screening, as well as screening suitable biomarkers for immune drug reactivity, thereby achieving precise treatment for clinical patients.

The current experimental objective of drug sensitivity screening of existing chemotherapeutic drugs or tumor-targeted drugs is tumor cells, which do not contain immune cells because most immune cells are removed. There is no screening method for drugs targeting immune cells or immune system regulation. Through numerous experiments, the present invention discloses a new animal model to detect the effects and mechanisms of immune drugs against tumors, inflammation, and infection by regulating the simulated immune system in the animal model, as well as screening suitable biomarkers for immune drug reactivity, thereby achieving rapid screening of immune system targeting or optimizing drug efficacy.

The present invention solves the above-mentioned technical problems through the following technical solutions:
A first aspect of the present invention provides a polyvinylidene fluoride (PVDF) capsule tube encapsulating cells, wherein the cells comprise tumor cells and immune cells in a single cell suspension, and further wherein the tumor cells and immune cells are derived from fresh tumor tissues or body fluids of clinical patients;
wherein the ratio of tumor cells to immune cells (CD3+T cell) ranges from 1.5: 1 to 15: 1, and/or,
wherein the percentage of CD8+ T cells in total live cells is above 0.5%;
wherein the immune cells are autologous immune cells enriched by sorting into a single cell suspension, and the tumor cells are those that flow through when sorting the single cell suspension, wherein the single cell suspension is derived from tumor-infiltrating lymphocytes in solid tumor tissue of a patient, and/or the peripheral blood mononuclear cells in peripheral blood of a patient.

In some embodiments of the present invention, the tumor tissues comprise clinically surgically resected tumor tissues or biopsied tumor tissues.

In some embodiments of the present invention, the body fluids comprise one or more of blood, bone marrow, pleural fluid and ascites and cerebrospinal fluid.

In some embodiments of the present invention, the average protein permeability of the polyvinylidene fluoride (PVDF) capsule tube is 300-1,000 kDa,or 500-700 kDa.

In some embodiments of the present invention, the polyvinylidene fluoride (PVDF) capsule tube is pre-processed by activating, flushing with ultrapure water and autoclaving before use.

In some embodiments of the present invention, the activating is accomplished by rinsing the polyvinylidene fluoride (PVDF) capsule tube in dehydrated alcohol.

In some specific embodiments of the present invention, the sorting method used in said sorting comprises sorting immune cells by adherence to magnetic beads conjugated with anti-human CD45.

In some specific embodiments of the present invention, the sorting method used in said sorting comprises sorting tumor cells by deleting immune cells and fibroblast cells using magnetic beads conjugated with anti-human CD45 and anti-human fibroblast cell markers.

In some specific embodiments of the present invention, the sorting equipment used in said sorting comprises:
(1) sorting magnetic beads, preferably anti-human CD45 microbeads, or anti-human CD45 microbeads and anti-human fibroblast microbeads;
(2) and the sorting device is preferably a LS column.

In some specific embodiments of the present invention, the cell suspension is prepared by the following steps:
(1) removing non-tumor tissue and necrotic tissue from the tumor sample, cutting the tumor sample into tissue blocks with a size of 1-2 mm, washing and centrifuging with HBSS buffer solution containing 1% BPS, and collecting the tumor tissue blocks;
(2) digesting with collagenase, terminating the digestion with a medium containing serum, and collecting the cell suspension; removing the supernatant by centrifugation and then collecting cell pellet;
(3) lysing red blood cells with 3 to 5 times the volume of erythrocyte-lysis solution, removing the supernatant by centrifugation, collecting the cell pellet, and repeating the erythrocyte lysis twice, wherein the erythrocyte lysis solution comprises 139.6 mmol/L NH4Cl, 16.96 mmol/L Tris pH 7.2;
   resuspending cell pellet in PBS with 1% FBS, removing the supernatant by centrifugation and collecting the cell pellet, and adjusting the cell concentration.

In some embodiments of the present invention, the ratio of tumor cells to immune cells (CD3+ T cell) ranges is from 1.5:1 to 9:1.

In some embodiments of the present invention, the percentage of CD8+ T cells in total live cells is from 0.5%- to 15%; and, the viability of the immune cells is more than 20%.

A second aspect of the present invention provides an animal model comprising at least one polyvinylidene fluoride (PVDF) capsule tube of the first aspect of the present invention, wherein the polyvinylidene fluoride (PVDF) capsule tube is implanted subcutaneously or orthotopically; the animal is a mouse.

In some embodiments of the invention, the mouse is an immunodeficient mouse selected from the group consisting of a BALB/c nude mouse, a NCG mouse, a NSG mouse, and a NOD-scid mouse.

In some embodiments of the present invention, the number of the polyvinylidene fluoride (PVDF) capsule tube is 1 - 8.

A third aspect of the present invention provides a method for rapid screening the efficacy of immunoregulating drugs, comprising the following steps:
(1) administering a drug to be tested to the animal model of the second aspect of the present invention; wherein the drug is an immunoregulating drug, wherein the drug is in a solid, semi-solid or liquid form; preferably, the drug is administered by intravenous injection, oral gavage, intraperitoneal injection or subcutaneous injection;
(2) detecting the total cell viability of tumor cells and immune cells or immune cell viability in the polyvinylidene fluoride (PVDF) capsule tube and the relative ratio and cell phenotype of tumor cells and immune cells; preferably, the detecting method of the total cell viability or immune cell viability is detected by the chemiluminescence method; and the detecting method of relative ratio and cell phenotype by flow cytometry (FACS);
(3) detecting molecular biological information of tumor cells and immune cells in the polyvinylidene fluoride (PVDF) capsule tube; preferably, the detection method of biological information is a DNA or RNA sequence; and wherein, steps (2) and (3) can be completed in any order; more preferably, the flow cytometry measures cellular expression of 7AAD, CD45, CD3, CD8, CD270, CD33, CD38, CD20, PDL1, PD1, CD68 and CD25.

Preferably, in step (2), the FACS analysis comprises one or more of the following steps:
antibody labeling, wherein the antibody comprises one or more of anti- 7AAD, CD45, CD3, CD8, CD270, CD33, CD38, CD20, PDL1, PD1, CD68 and CD25;
performing flow cytometry detection, wherein PBS with 2% FBS is added after said antibody labeling is completed and then loaded for detection in the FACS; and
data analysis by software, wherein the software is preferably Flowjo.

A fourth aspect of the present invention provides a use of the capsule tube of the first aspect of the present invention for the preparation of an animal model for rapid screening of the efficacy of an immunoregulating drug; the animal is an immunodeficient mouse; preferably, capsules of more than one patient are simultaneously implanted and each capsule contain cells that can be derived from different patients.

In some specific embodiments of the present invention, the animal model can perform simultaneous drug screening for 1-8 patients, preferably 1-4 patients.

The above preferred conditions can be combined arbitrarily to obtain the preferred embodiments of the present invention based on the common knowledge in the art.

The reagents and raw materials used in the present invention are all commercially available.

The positive progressive effect of the present invention is that:

the present invention performs immune system targeting and optimizes drug screening by constructing an animal model using a capsule tube. The models and methods provided by the present invention provide rapid screening methods for the sensitivity and effectiveness of immune drugs for clinical patients, obtaining pharmacodynamic data from the animal model in vivo, phenotypic identification data of paired flow multicellular subgroups and multi-omics analysis data, and provides rapid and effective functional and mechanistic information for clinical precision medicine and new drug development.

### BRIEF DESCRIPTION OF THE DRAWINGS

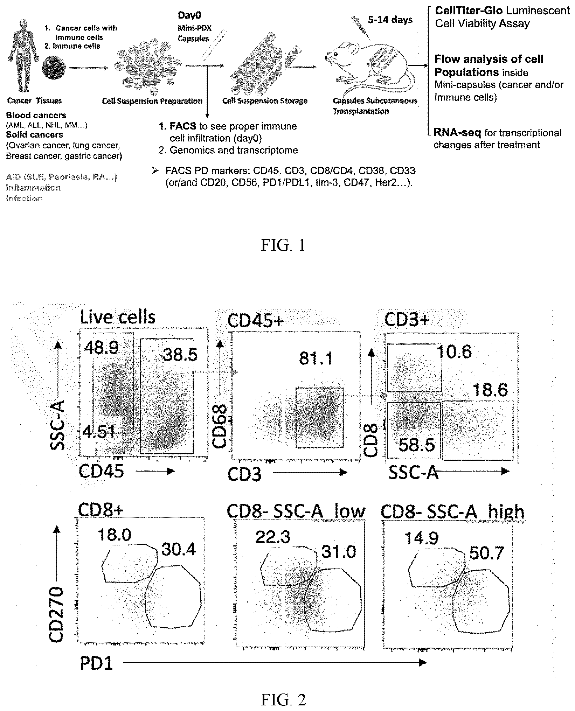

FIG.1 is a schematic diagram of a method for rapid screening of immune drugs by "IO-FIVE (Immuno-Oncology fast In Vivo Efficacy test)" technology. Tumor immunotherapy is taken as an example. Tumor tissues derived from a patient are taken and processed to make the tumor cells and autologous immune cells mixed in a certain ratio (recommended 1.5:1 to 15:1) and the mini-capsule tubes are sealed, and the immunodeficient mice are inoculated subcutaneously or orthotopically and administered drug. After 5 to 14 days, the mini-capsule tubes are taken out for the detection of total cell viability, the ratio of cellular subgroups, cell phenotypes as identified by flow cytometry (FACS), and various omics (e.g., transcriptome) are analyzed before and after drug administration. In the case of the autoimmune diseases, immune cells were obtained from inflammatory tissues (such as lymph nodes) and peripheral blood of the patient for detecting cell phenotype by FACS.
FIG. 2 is shows the phenotype detection of single cell suspension (in the capsule tubes, i.e. mini-capsules) of tumor tissues by flow cytometry on day 0 for a patient with ovarian cancer. (Example 1)
FIG. 3 shows the detection of the viability of total cells (containing tumor cells and immune cells) in the capsule tubes on day 10 by CTG (CellTiter-Glo) chemiluminescence method for the patient with ovarian cancer. (Example 1)
FIG. 4 shows the phenotype analysis of the single cell suspension in the capsule tubes on day 10 by flow cytometry (FACS) for the patient with ovarian cancer. (Example 1)
FIG. 5 shows the phenotype detection of the single cell suspension of tumor tissues (in the capsule tubes, i.e., mini-capsule tubes) by flow cytometry on day 0 for a patient with ovarian cancer. (Example 2)
FIG. 6 shows the detection of the viability of total cells (containing tumor cells and immune cells) in the capsule tubes on day 10 by CTG method for the patient with ovarian cancer. (Example 2)
FIG. 7 shows the phenotype detection of tumor tissues (tumor cells and immune cells) in the mini-capsule tubes on day 10 by FACS for the patient with ovarian cancer. (Example 2)
FIG. 8 shows the phenotype detection of different types of single cell suspension of tumor tissues (tumor tissues before purification, tumor cells after purification, immune cells after purification, tumor samples after addition of immune cells) on day 0 by flow cytometry for a patient with ovarian cancer. (Example 3)
FIG. 9 shows the detection of the viability of total cells (containing tumor cells and immune cells) in the capsule tubes on day 10 by CTG method for the patient with ovarian cancer (tumor tissues before purification, and tumor samples after addition of immune cells). (Example 3)
FIG. 10 shows the phenotype detection of tumor tissues (tumor cells and immune cells) in each mini-capsule tubes by FACS on day 10 for the patient with ovarian cancer. (Example 3)
FIG. 11 shows the phenotype detection of single cell suspension of tumor tissues (in mini-capsule tubes) on day 0 by flow cytometry for a patient with lung cancer. (Example 4)
FIG. 12 shows the detection of the viability of total cells (containing tumor cells and immune cells) in the capsule tubes on day 14 by CTG method for the patient with lung cancer. (Example 4)
FIG. 13 shows the phenotype detection of tumor tissues (tumor cells and immune cells) in the mini-capsule tubes on day 14 by FACS for the patient with lung cancer. (Example 4)
FIG. 14 shows the phenotype detection of single cell suspension (in the mini-capsule tubes) of tumor tissues (peripheral blood) on day 0 by flow cytometry for two AML patients. (Example 5)
FIG. 15 shows the detection of the viability of total cells (containing tumor cells and immune cells) in the mini-capsule tubes on day 14 by CTG method for two AML patients. (Example 5)
FIG. 16 shows a. the phenotype detection of tumor tissues (tumor cells and immune cells) in the mini-capsule tubes by FACS on day 14 for an AML patient of immunoreactive type 124#. b. the analysis of the ratio of CD8+T in live cells on day14 by FACS for two AML patients, 124# (immunoreactive type) and 123# (immune nonreactive type). (Example 5)
FIG. 17 shows a. the heat-map showing transcriptomic differential gene signaling pathways of the two AML patients on day 0; b. comparative analysis of tumor proliferation level, angiogenesis level, neutrophil activity level and effector (T) cell level biomarkers for two AML patients with differential gene expression of clusters. (Example 5)
FIG. 18 shows the analysis differential expression of key membrane protein molecules in genes for two AML patients. (Example 5)
FIG. 19 shows genomic DNA analysis including genomic CNV and mutation for two AML patients. (Example 5)
FIG. 20 shows the phenotype detection of single cell suspension (in the mini-capsule tubes) of tumor tissues (peripheral blood, bone marrow in combination with peripheral blood) on day 0 by flow cytometry for three AML patients. (Example 6)
FIG. 21 shows the detection of the viability of total cells (containing tumor cells and immune cells) in the capsule tubes on day 14 by CTG method for three AML patients. (Example 6)
FIG. 22 shows the phenotype detection of tumor tissues (tumor cells and immune cells) in the mini-capsule tubes by FACS on day 14 for three patients. (Example 6)
FIG. 23 shows the phenotype detection of single cell suspension of tumor tissues (bone marrow in combination with peripheral blood) on day 0 by flow cytometry for an AML patient. (Example 7)
FIG. 24 shows the detection of the viability of total cells (containing tumor cells and immune cells) in the mini-capsule tubes on day 14 by CTG method for an AML patient. (Example 7)
FIG. 25 shows (Example 7) the detection of the ratio of tumor tissues (tumor cells and immune cells) in the mini-capsule tubes on day 14 by FACS for a patient.
FIG. 26 shows (Example 8) the phenotype detection of single cell suspension (in mini-capsule tubes) of tumor tissues (bone marrow) on day 0 by flow cytometry for two AML patient.
FIG. 27 shows (Example 8) the detection of the viability of total cells (containing tumor cells and immune cells) in the capsule tubes on day 10 by CTG method for two AML patients, including two experiments: (1) the experiment that the same mouse is inoculated with the mini-capsule tubes containing the cells from the two patients (two patients in one mouse) and (2) the classical experiment that one mouse is inoculated with the mini-capsule tubes containing the cells from one patient (one patient in one mouse).
FIG. 28 shows (Example 8) the detection of the ratio of tumor tissues (tumor cells and immune cells) in the mini-capsule tubes on day 10 by FACS for two patients.

### DETAILED DESCRIPTION OF THE PREFERRED EMBODIMENT

The present invention is further illustrated by way of the following examples, but the invention is not limited to the scope of the described examples. The experimental methods for which specific conditions are not specified in the following examples are chosen according to conventional methods and conditions, or according to the descriptive literature.

### Example 1 Rapid functional screening and potential biomarker identification of immunotherapy for a patient with ovarian cancer

### 1. Experimental animal preparation and sample collection

BALB/c nude mice (available from Jiangsu Jicui Yaokang Biotechnology Co. Ltd.) aged 6-8 weeks were ordered from the supplier in this experiment and kept in the animal house at SPF level, and the animals were adapted for at least 3 days before the start of the experiment.

Fresh tumor samples from the patient were collected and placed in anticoagulant tubes and transported to the central laboratory by cold chain (2-8°C) within a short period of time.

### 2. Cell processing

Non-tumor tissue and necrotic tissue were removed from tumor samples of patients, tumor samples were cut into tissue pieces of 1-2 mm in size, washed and centrifuged with HBSS buffer containing 1% PSB (Penicillin and streptomycin + Amphotericin B), and tumor tissue pieces were collected. Digestion was performed using lx collagenase for 1-2 h at 37 °C. Said sorting method was as follows:
The digestion was terminated by serum-containing medium (RPMI 1640, available from Gibco) and the cell suspension was collected with a 70 µM strainer. The supernatant was removed by centrifugation at 1000 rpm for 5 min and the cell pellet was collected. 3-5x lysis solution (139.6 mmol/L NHCl, 16.96 mmol/L Tris, adjusted to pH 7.2 with 1 mol/L HCl) was used to lyse red blood cells at 4°C for 5 min, the supernatant was removed by centrifuge at 1000 rpm for 5 min. The cell pellet was collected, and lysis of erythrocyte was repeated twice. The cell pellet was resuspended in PBS with 1% FBS, the supernatant was removed by centrifugation at 1000 rpm for 5 min, the cell pellet was collected, and the cell concentration was adjusted to 1 x 108 cells/ml.

Anti-human CD45 microbeads were added or anti-human CD45 microbeads and anti-human fibroblast microbeads were added at the concentration of 20 µl/10⁷ cells, and incubated at room temperature for 30 min. PBS with 1% FBS was added to rinse the magnetic beads. The supernatant was removed by centrifuge at 1000 rpm for 5 min, and PBS with 1% FBS was used to resuspend the cell pellet. The magnetic beads were loaded on a sorting device (LS column, Miltenyi Biotec). The LS column was washed twice with PBS with 1% FBS. The liquid flowing down from the LS column (tumor cells) and the cells attached to the LS column (enriched immune cells) were collected respectively. The collected cell suspension was centrifuged at 1000 rpm for 5 min to remove the supernatant. The cells were, resuspended in cell culture medium (available from Gibco) and counted.

In general, tumor tissues need to be purified by adding two both anti-human CD45 microbeads and anti-human fibroblast microbeads, so as to preserve the immune cells and stromal fibroblasts from the microenvironment of tumor tissues. However, when the infiltration of immune cells in some tumor tissues was excessive and more than half of the ratio, such as metastatic samples of pleural fluid and ascites of solid tumor, CD45 antibody microbeads were added to enrich immune cells.

### 3. Flow cytometry detection and data analysis

A single cell suspension of tumor tissue samples of patients before purification, a single cell suspension of tumor cells after purification, and a single cell suspension of enriched immune cells (containing CD3 + T) were taken respectively. They were then mixed into a suspension of tumor cells and immune cells of different ratios (1.5:1 to 15:1), each containing about 0.1 to 0.3 million cells. For each sample, the suspension was divided into multiple portions according to the purpose of the experiment, and the corresponding flow cytometry antibody mixture was added. The experimental group and control was set up using direct labeling and incubated at 2-8°C for 25-30 min in darkness. The preliminary FACS panel for ovarian cancer was labeled with antibodies to 7AAD, CD45, CD3, CD8, CD270, PD1, CD68 and CD25.

After completion of antibody labeling, PBS with 2% FBS was added. The cells were centrifuged at 600 g for 5 min at 4°C to remove the supernatant. The cell pellet was resuspended by adding 250 µl of 2% FBS (available from Gibco) in PBS and loaded for flow cytometry detection. Data analysis for flow cytometry detection was performed by Flowjo software.

### 4. Cell tubing

The capsule device was taken from the biological safety cabinet, i.e., PVDF tube with diameter of 1-2 mm, wherein the average pore size was about 500 KDa molecular weight. The PVDF tube was subjected to anhydrous ethanol activation (or activation with methanol and the like), ultra-pure water flushing, and autoclaving and the like before being filled with the cell suspension. A capsule tube of 1.5 cm in length was taken and washed repeatedly with cell culture medium (available from Gibco).

Using flow cytometry, the CD45-tumor cells and CD3 + lymphocytes were in a certain ratio (1.5:1 to 15: 1). The CD8 + T cells accounted for more than 0.5% of total living cells. The cell suspension was put into PVDF tubes and sealed.

### 5. Inoculation and drug administration

The above PVDF tubes were inoculated into mice subcutaneously with a puncture needle. The wounds were sealed with medical tissue glue (available from 3M). The control groups and drug administration groups were chosen (such as Group 1. Paclitaxel, cisplatin and Avastin/Bevacizumab; Group 2. PD1 antibody; Group 3. PD1 antibody combined with the PARP inhibitor Niraparib; Group 4. Niraparib alone), and then the drugs were administered to the groups.

### 6. Cell viability and phenotype detection

3-21 days after administration (day 10 as shown), the mice were euthanized, and the PVDF tubes were removed. Cell viability was quantified using the CellTiter-Glo chemiluminescence method, and the relative ratio and phenotype of tumor cells and immune cells in PVDF tubes were detected by flow cytometry. The changes in the molecular biological information from the tumor cells and immune cells in PVDF tubes were detected by molecular biological methods (RNA sequencing, and DNA sequencing technology).

### 7. Data analysis

Flow cytometry (FACS) screening on day 0 was as follows. The relative ratio and cell phenotype of primary tumor cells and immune cells of mixed cells in capsule tubes indicated that CD45-tumor cell ratios were: CD45 + to CD3 + T immune cells equaled 1.6:1, CD8 +% equaled 3.1%, and T lymphocytes inside the tumor, especially CD8-activated cells, highly expressed PD1 molecules, while CD8 + T cells also weakly expressed both CD270 and PD1 immune checkpoint molecules, as shown in FIG. 2.

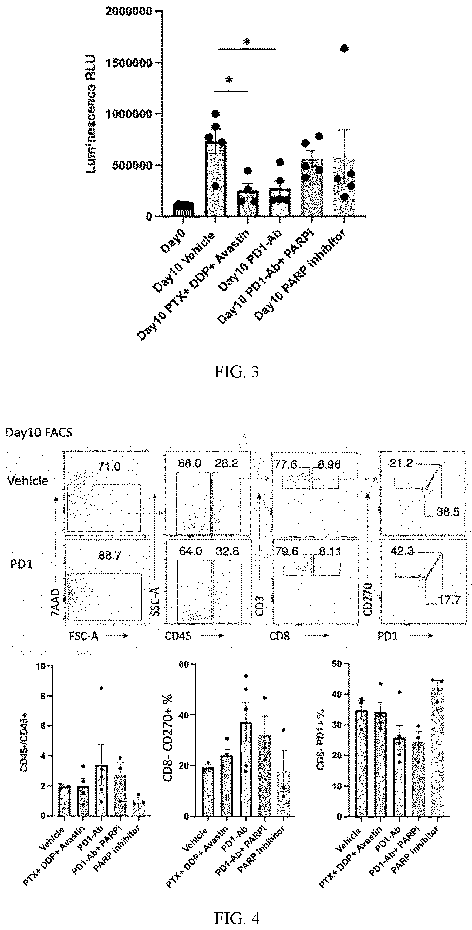

The control endpoint of experiments in vivo on day 10 was as follows. Based on the the viability of the cells in the control group (as shown in FIG. 3), the proliferation viability of all cells in the drug administration group was calculated, and the efficacy of the drug was calculated.

The experimental endpoint in vivo on day 10 was as follows. The relative ratio and cell phenotype of tumor cells and immune cells in PVDF tubes were detected by flow cytometry (as shown in FIG. 4). The results were analyzed by Flowjo software. CTG and FACS detection on day 10 showed that PD1 antibody could effectively motivate autoimmune cells of the patients to inhibit the proliferation and metabolism of the tumor cells of ovarian cancer, whether in PD1 mono-therapy group or in combo-therapy with PARP inhibitor group. CD270 molecule (HVEM) was up-regulated to some extent along with the blocking and down-regulation of PD1 molecules by PD1 antibody, whether in PD1 mono-therapy group or in combo-therapy with PARP inhibitor group.

Changes in the molecular biological information of tumor cells and immune cells in PVDF capsule tubes in clinical samples on day 0 or day 10 were detected by molecular biological methods (DNA sequencing, RNA sequencing technology and other "omics" technologies).

Therefore, the present invention provides a method for the rapid screening of immune drugs by "IO-FIVE" (Immuno-Oncology Fast In Vivo Efficacy test) technology, which is also used in the following examples.

### Example 2 Rapid functional screening and potential biomarker identification of immunotherapy for a patient with ovarian cancer

### 1. Experimental animal preparation, sample collection, cell processing, flow cytometry detection and data analysis methods, and cell sealing requirements were the same as in Example 1.

### 2. Inoculation and drug administration

As per above PVDF tubes were inoculated into mice subcutaneously with a puncture needle, the wounds were sealed with medical tissue glue (available from 3M), and control groups and drug administration groups were randomly set (such as Group 1. Paclitaxel, cisplatin and Bevacizumab combination therapy; Group 2. PD1 antibody; Group 3. PD1 antibody combined with Bevacizumab; Group 4. PD1 antibody combined with cisplatin), and the drugs were administered.

### 3. Cell viability and phenotype detection

3-21 days after administration (day 10 here), the mice were euthanized, and the PVDF tubes were removed. Cell viability was quantified by the CellTiter-Glo chemiluminescence method, and the relative ratio and basic phenotype of tumor cells and immune cells in PVDF tubes were detected by flow cytometry.

### 4. Data analysis

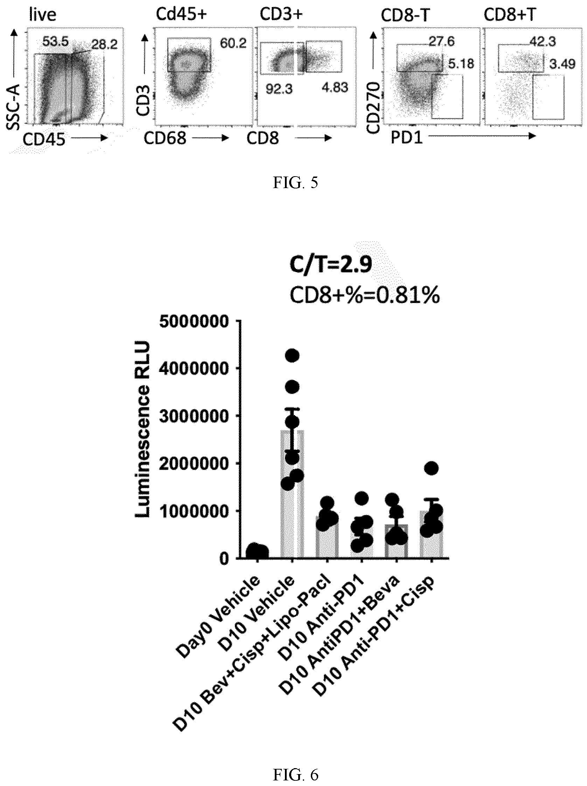

Flow cytometry (FACS) screening on day 0 was as follows. the relative ratio and cell phenotype of primary tumor cells and immune cells, flow cytometry data of mixed cells in capsule tubes indicated that the ratio of CD45- tumor cells to CD45+CD3+ T immune cells was about 2.91:1, and the CD8+ cell percentage was 0.81% for living cells. Infiltrating T lymphocytes inside the tumor weakly expressed PD1 molecules, but, significantly, also highly expressed CD270 (as shown in FIG. 5).

The control endpoint of experiments in vivo on day 10 was as follows. According to the viability value of the cells in the control group (as shown in FIG. 6), the proliferation viability of all cells in the drug administration group was calculated, and then the efficacy of the drug to be tested was calculated.

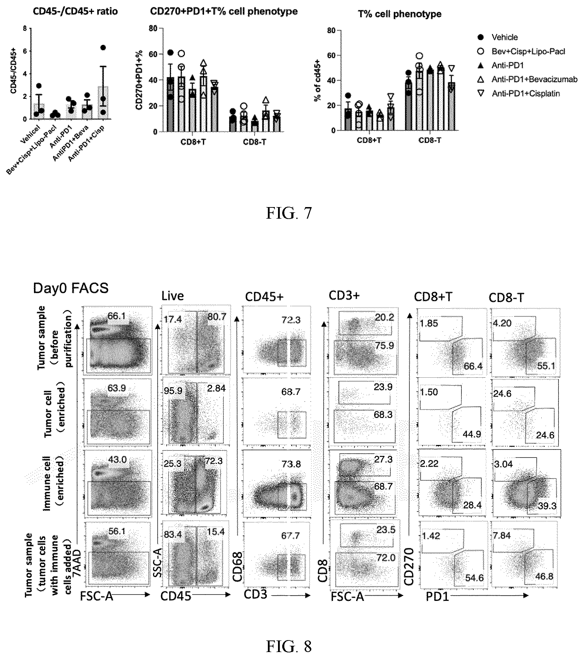

The experimental endpoint of experiments in vivo on day 10 was as follows. The relative ratio and cell phenotype of tumor cells and immune cells in PVDF tubes were detected by flow cytometry (as shown in FIG. 7). The results were analyzed by Flowjo software.

Based on the experimental results shown in FIG. 6 and FIG. 7, at least half of the live cells in the capsule tubes on day 10 were CD45- tumor cells. Compared with control group, PD1 monotherapy group (except the combo-therapy group of paclitaxel, cisplatin and Bevacizumab, or combo-therapy group with targeting drug Bevacizumab or combo-therapy group with chemical drug cisplatin) could significantly inhibit the overall viability of tumor tissues (including tumor cells and immune cells). CD270 +, PD1 + double-positive TIL were enriched in the mini-capsules along with the progression of the tumor, which showed a trend of down-regulation in the PD1 mono-therapy group. Combined with the FACS data typing in Example 1, these data indicated that CD270 molecule was efficiently expressed in solid tumors, which was likely a positive biomarker for use of immune checkpoint inhibitor PD1 antibody therapy. Therefore, even patients with weak initial PD1 expression and significant CD270 expression would likely still respond well to immunotherapy.

Changes in the molecular biological information of tumor cells and immune cells in PVDF capsule tubes in clinical samples on day 0 or day 10 were detected by molecular biological methods (DNA sequencing, RNA sequencing and other "omics" technologies).

### Example 3 Rapid functional screening of immunotherapy for a patient with ovarian cancer

Experimental animals, sample collection, and cell processing were the same as in Example 1.

### 2. Flow cytometry detection and data analysis

The following was collected for cell identification: a single cell suspension of tumor tissue samples of patients before purification, tumor cells after purification, enriched immune cells (containing CD3 + T) eluted from the column, and a cell suspension in which tumor cells after purification and immune cells were mixed in a certain ratio (1.5:1 to 15:1), to yield a final cell count of 0.1 to 0.3 million cells. (Note that if the viability of immune cells eluted from the column was less than 20%, then downstream experiments and operations could not be performed, especially since activated immune cells were more likely to undergo activation-induced apoptosis). Then, each sample suspension was divided into multiple portions according to the purpose of the experiment, and the corresponding flow cytometry antibody mixture was added, and the corresponding experimental group with control was set up, including direct labeling, and incubated at 2-8°C for 25-30 min in darkness. The FACS panel for ovarian cancer were as follows: Antibodies to 7AAD, CD45, CD3, CD8, CD270, PD1, CD68 or CD25. After completion of antibody labeling, PBS with 2% FBS was added, and the cells centrifuged at 600 g for 5 min at 4°C. The supernatant was removed, and the cell pellet was resuspended by adding 250 µl of 2% FBS (available from Gibco) in PBS and loaded for flow cytometry detection. Data analysis for flow cytometry detection was performed by Flowjo software.

### 3. Cell tubing

The basic operational steps were the same as those in Example 1.

According to the data analyzed in step of flow cytometry, the CD45- tumor cells and CD3+ T cells were mixed in a certain ratio (about 1.5:1-15:1). CD8+ T cells accounted for more than 0.5% of total live cells. The suspension was filled into PVDF tubes and sealed. In this example, the tumor samples before purification were also used for controls for tube sealing and other experiments.

### 4. Inoculation and drug administration

As above, the PVDF tubes were inoculated into mice subcutaneously with a puncture needle, and the wounds were sealed with medical tissue glue (available from 3M), and control groups and the drug administration groups were randomly set (Group 1. Paclitaxel, cisplatin and Bevacizumab combo-therapy, Group 2. PD1 antibody; group 3. PD1 antibody combined with PARP inhibitor Niraparib; Group 4. PARP inhibitor Niraparib), and the drugs were administered to the mice.

### 5. Cell viability and phenotype detection

3-21 days after administration (day 10 here), the mice were euthanized, and the PVDF tubes were removed. Cell viability was quantified by the CellTiter-Glo chemiluminescence method, and the relative ratio and basic phenotype of tumor cells and immune cells in PVDF tubes were detected by flow cytometry.

### 6. Data analysis

Flow cytometry (FACS) screening on day 0 results for the relative ratio and cell phenotype of primary tumor cells and immune cells: (1) In the tumor tissue before purification, the ratio of tumor cells CD45- and CD3 + T cells (C/T) was 0.3, and the CD8 + T cell percentage was 11.5, (i.e. the tumor sample of the patient was rich in TILs); (2) Tumor tissue cells after purification, contained only 2.84% immune cells, with a C/T value of 49.1, and CD8 + cells were 0.448%; (3) The total viability of the immune cell population after purification was about 43%, which retained the basic cell phenotype before purification. (4) Tumor tissue samples after adding immune cells had a C/T ratio of 8 and a CD8 + percentage of 2.45%. The infiltrating T lymphocytes inside the tumor weakly expressed CD270 molecules and highly expressed PD1 molecules, indicating a better response to PD1 antibodies (see FIG. 8).

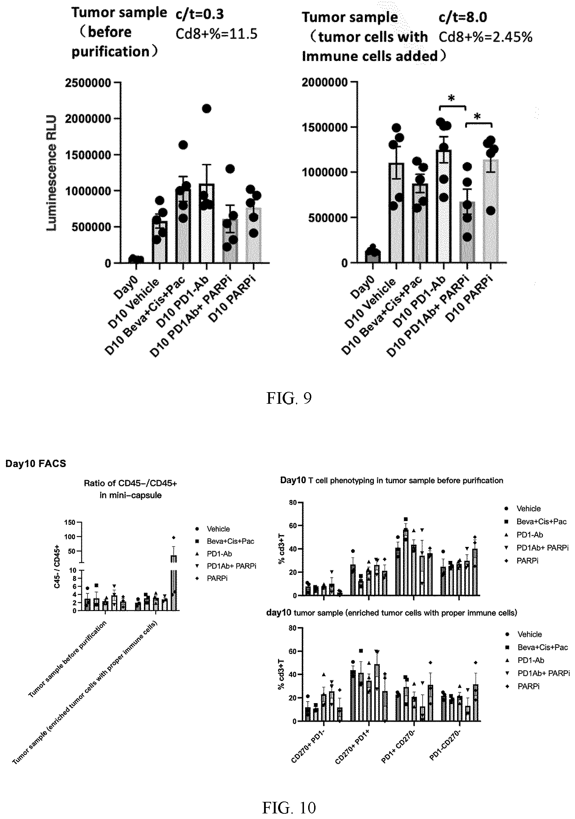

Control results in vivo on day 10: adjusted to the viability value of the cells in the control group (as shown in FIG. 9), the proliferation viability of all cells in the drug administration group was calculated, and then the efficacy of the drug to be tested was calculated.

Experiment results in vivo on day 10: the relative ratio and cell phenotype of tumor cells and immune cells in PVDF tubes were detected by flow cytometry (as shown in FIG. 10). The results were analyzed by Flowjo software.

Based on the experimental results shown FIG. 9 and FIG. 10 for the mini-capsules of day 10, whether before purification or autologous TILs added to the sample, the tumor cells were mainly CD45- cells, which were present at about 2-3 times of the CD45 + cells (FIG. 10); CTG data of tumor tissue samples before purification indicated that a drug treatment including clinical first-line drugs would not significantly reduce the viability of total cells of the tissue sample. However, the response of the tumor sample after addition of autologous TIL cells, indicated a clinical first-line drug combination could weakly reduce the viability of total cells of the sample, although neither PD1 antibody nor PARP inhibitor mono-therapy could inhibit the overall tumor viability. The combination of PD1 antibody and PARP inhibitor could significantly inhibit the overall tumor proliferation and activity (FIG. 9). Interestingly, the combination of PD1 antibody and PARP inhibitor significantly increased the ratio of CD270+, PD1- T cell subgroup and relatively reduced the ratio of PD1 +, CD270- to PD1-, CD270-T cells (FIG. 10), indicating that CD270 molecule may be a potential biomarker for PD1 antibody therapy.

### Example 4 Rapid functional screening of immunotherapy for a patient with lung cancer

### 1. The experimental animals, sample collection and cell processing were the same as in Example 1.

### 2. Flow cytometry detection and data analysis

A single cell suspension of tumor tissue samples of patients was made before purification. Tumor cells after purification and enriched immune cells were collected respectively, and then mixed in a suspension. The tumor cells and CD3+ T cells were prepared are different ratios (1.5:1-15:1), with each sample containing about 0.1-0.3 million cells. Each sample suspension was divided into multiple portions according to the purpose of the experiment, and the corresponding flow cytometry labeling antibody was added, and the corresponding experimental group with control was set up, such as for direct labeling. The sample was incubated at 2-8°C for 25-30 min in darkness. The FACS panel for lung cancer included 7AAD, CD45, CD3, CD8, etc. After completion of antibody labeling, PBS with 2% FBS was added, and centrifuged at 600 g for 5 min at 4°C, and then the supernatant was removed. The cell pellet was resuspended by adding 250 µl of 2% FBS (available from Gibco) in PBS and loaded for flow cytometry detection. Data analysis for flow cytometry detection was performed by Flowjo software.

### 3. Cell tubing

The capsule device was taken from the biological safety cabinet. The PVDF tube had a diameter of 1-2 mm, and the average pore size was about 500 KDa molecular weight. The PVDF tube was subjected to anhydrous ethanol activation, ultra-pure water flushing and autoclaving and the like before the cell suspension was used to fill the capsule. A capsule tube of 1.5 cm in length was taken and washed repeatedly with cell culture medium (available from Gibco).

Based on the to flow cytometry results, the tumor cells and CD3 + lymphocytes were mixed well in a certain ratio (1.5:1-15:1). The CD8 + T cells accounted for more than 0.5% of total living cells. The suspension was used to fill the PVDF tubes and sealed.

### 4. Inoculation and drug administration

The above PVDF tubes were inoculated into mice subcutaneously with a puncture needle, and the wounds were sealed with medical tissue glue (available from 3M). The control groups and drug administration groups were randomly set (such as Group 1. PD1 antibody; Group 2. antibody combined with HDAC inhibitor Chidamide), and the drugs were administered.

### 5. Cell viability and phenotype detection

3-21 days after administration (day 14 here), the mice were euthanized, and the PVDF tubes were removed. Cell viability was quantified by the CellTiter-Glo chemiluminescence method, and the relative ratio and basic phenotype of tumor cells and immune cells in PVDF tubes were detected by flow cytometry.

### 6. Data analysis

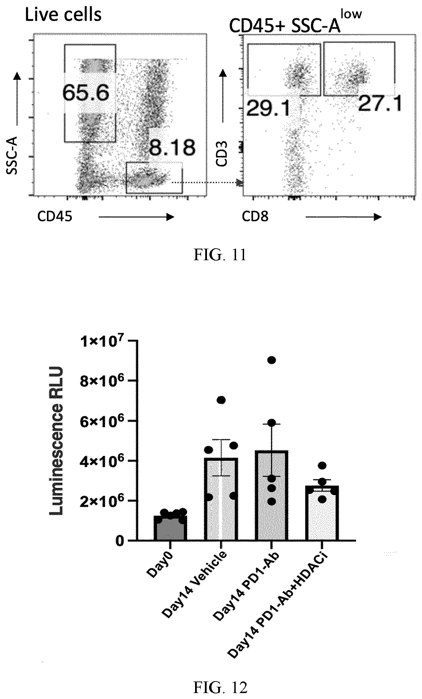

Flow cytometry (FACS) results on day 0 for the relative ratio and cell phenotype of primary tumor cells and immune cells were as follows. Flow cytometry data of mixed cells in capsule tubes indicated that CD8 + cells were 3.1%. The ratio of CD45- tumor cells to CD45+, CD3+ T immune cells was about 15:1, and CD8+ were 2.2% (as shown in FIG. 11).

Experiment results in vivo on day 14 were are follows. Based on to the viability value of the cells in the control group (as shown in FIG. 12), the proliferation and viability of all cells in the drug administration group was calculated, and the efficacy of the drug was calculated.

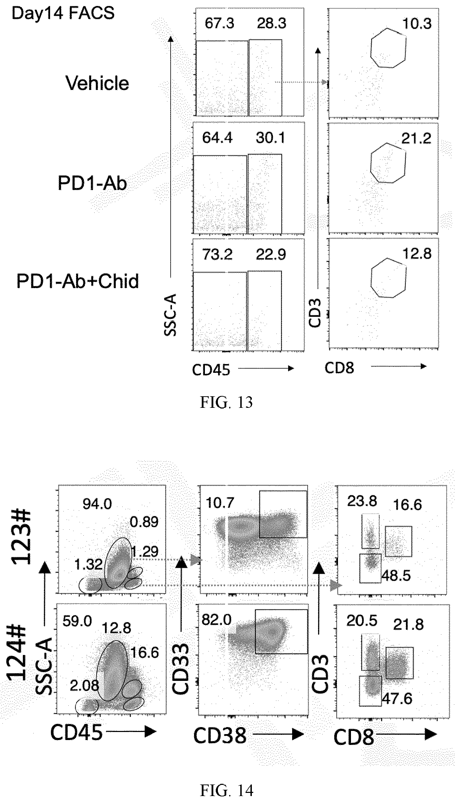

Experiment results in vivo: the relative ratio and cell phenotype of tumor cells and immune cells in PVDF tubes were detected by flow cytometry (as shown in FIG. 13). The results were analyzed by Flowjo software.

Based on the experimental results shown in FIG. 12 and FIG. 13, CD45- tumor cells were dominant in the capsule tubes on day 14, at 60-75%. PD1 antibody treatment did not down-regulate the viability of total cells of tumor cells and immune cells, although it promoted the survival of CD8+ T cells. When PD1 antibody was combined with HDAC inhibitor Chidamide, the total viability of total cells (ATP activity) was significantly reduced. Therefore, the patient with lung cancer was not suitable for PD1 antibody monotherapy, but combo-therapy of immunotherapy combined with chemically targeting drugs was recommended.

### Example 5 Rapid functional screening and potential biomarkers identification of immunotherapy for two patients with acute myeloid leukemia AML

### 1. Experimental animals and sample collection

BALB/c Nude mice (available from Jiangsu Jicui Yaokang Biotechnology Co. Ltd.) aged 6-8 weeks were ordered from the supplier in this experiment, and kept in the animal house at SPF level, and the animals were adaptively kept for at least 3 days before the start of the experiment.

Fresh peripheral blood samples from two acute myeloid leukemia (AML) patients (in the acute phase of AML) were collected, and placed in anticoagulant tubes, and transported to the central laboratory by cold chain (2-8°C within a short period of time.

### 2. Cell processing

The peripheral blood samples of different patients were transferred into the biological safety cabinet. The cells were centrifuged using gradient centrifugation gel (available from SIGMA) with a density of 1.077 g/mL at room temperature, two new centrifugal tubes were added, two peripheral blood samples of patients were respectively added into one of the centrifugal tubes, and the above-mentioned liquid was slowly placed into the centrifuge, with the temperature set at 20-25°C (room temperature) with increasing and decreasing speed at 1 or 0 at 400 g for 20 min. After centrifugation, the centrifugal tubes were slowly moved, and the middle layer of cloudy cell layer (containing a large number of mononuclear cells) was collected, washed with 15 ml PBS (available from Gibco), and subjected to centrifugation at 1500 rpm for 5 min and the supernatant removed, and the normal cell suspension was subjected to further centrifugal washing. If necessary, 3-5x of erythrocyte lysis solution (available from Invitrogen) was used to lyse red blood cells. The cells were washed for 5 min at 1500 rpm and the supernatant removed. After resuspending in cell culture medium (available from Gibco), the cells were counted.

### 3. Flow cytometry detection and data analysis

0.3 million peripheral blood cells were taken respectively, and subjected to staining and sorting by selecting different flow cytometry antibodies according to the experimental purposes, incubated 25-30 min at 2-8°C in darkness for antibody labeling. The FACS panel commonly used in AML patients was: 7AAD, CD45, CD3, CD8, CD33, CD38, CD20, CD25 or PD1. After completion of antibody labeling, PBS with 2% FBS was added, and centrifuged at 600 g for 5 min at 4°C and the supernatant removed. The cell pellet was resuspended by adding 250 µl of 2% FBS (available from Gibco) in PBS and loaded into the flow cytometer. Data analysis for flow cytometry was performed using Flowjo software.

### 4. Cell tubing

The capsule device was taken from the biological safety cabinet. The PVDF tube had a diameter of 1-2 mm (the average pore size of the material was about 300 kDa-700 kDa, allowing the ingress and egress of macromolecular proteins and the like, but not allowing free ingress and egress of cells). PVDF tube was subjected to anhydrous ethanol activation, ultra-pure water flushing and autoclaving and the like before being filled with the cell suspension. A capsule tube of 1.5 cm in length was taken and washed repeatedly with cell culture medium (available from Gibco).

Quality control analysis was performed according to the flow cytometry analysis in step (3), so as to meet the requirement that the ratio of CD8+ T cells was at least more than 0.5% (the basic value of action of the immune drug). Then, the tumor cells (or non-CD 45high SSC-Alow cells) and the CD45high SSC-AlowCD3+ T lymphocytes were uniformly mixed to a certain ratio (1.5:1 to 15:1), and then filled into PVDF tubes and sealed.

### 5. Inoculation and drug administration

The above PVDF tubes were inoculated into mice subcutaneously with a puncture needle, and the wounds were sealed with medical tissue glue (available from 3M). The control groups and drug administration groups were randomly set (such as PD1 antibody, CD38 antibody, Daratumumab, Bcl2 inhibitor Venetoclax, HDAC inhibitor Chidamide in mono-therapy or combo-therapy), and the drug administered to the groups.

### 6. Cell viability and phenotype detection

3-21 days after administration (such as day 14), the mice were euthanized, and the PVDF tubes were removed. Cell viability was quantified by the CellTiter-Glo chemiluminescence method. Changes of the relative ratio and phenotype of tumor cells and immune cells in PVDF tubes were detected by flow cytometry; and changes in molecular biological information of tumor cells and immune cells in PVDF capsule tubes of clinical samples of day 0 or day 10 were detected by molecular biological methods (RNA sequencing, and DNA sequencing technology, etc.).

### 7. Data analysis

Flow cytometry results on day 0: the relative ratio and cell phenotype of primary tumor cells and immune cells for the two AML patients (#123, #124) are shown in FIG. 14. The CD8+ T cells accounted for 0.21% of all living cells (it is speculated that this patient may not be sensitive to PD1 antibodies) and C/T (the ratio of CD45lowCD33 + CD38 + tumor cells to CD3 + T) was about 15:1 (more CD38- tumor cells were also present) in patient 123#, while the CD8+ T cell percentage was about 3.48% and the C/T value was about 6: 1 in patient 124#.

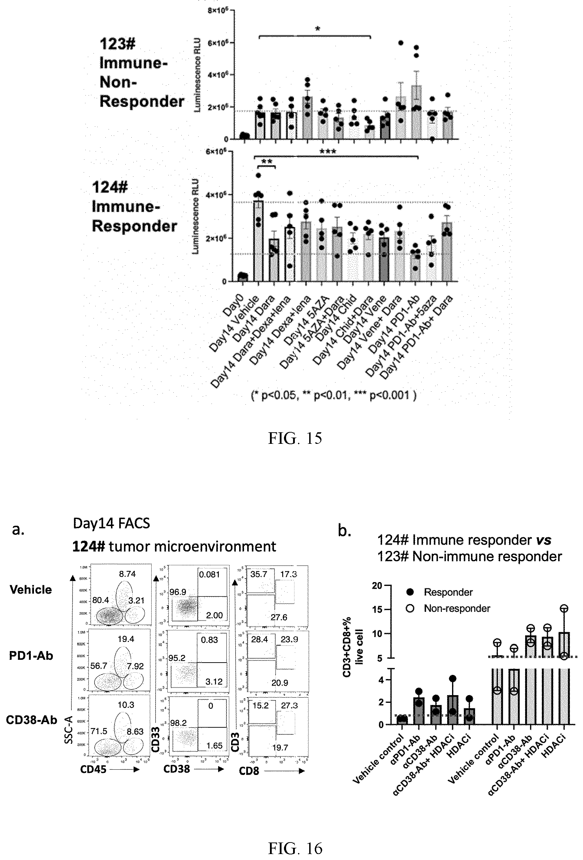

CTG viability on day 14: according to the viability value of the cells in the control group (as shown in FIG. 15), the proliferation viability of all the cells in the drug administration group was calculated, and then the efficacy of the drug was calculated.

Flow cytometry detection on day14: the relative ratio of tumor cells and immune cells in PVDF tubes were detected by flow cytometry; the differences between tumor cells and immune cells in the drug group and the control group were compared (as shown in FIG. 16). The results were analyzed by Flowjo software.

Based on the above data analysis of CTG and FACS on day 14, the living cells in the capsule tubes of patients 123# and 124# were still accounted for. The vast majority were CD45-/low tumor cells (such as AML cancer blasts), and about 5%-10% were CD45highSSC-Alow lymphocytes (124# as an example shown in FIG. 16a). Patient 124 (Immune Responder) showed a good response to the check point inhibitor PD1 antibody (more than half of the CTG inhibitory effect in FIG. 12), which coincided with the high level of CD8+ T cells on day 0, as well as a significantly reduced ratio of AML blasts in living cells on day14 (FIG. 16b), and a relatively increased ratio of CD3+ CD8+ (FIG. 16b). This phenomenon did not appear in patient 123# (Immune Non-responder). In addition, CD38 antibodies with antibody-dependent cytotoxicity (ADCC) function and antibody-dependent macrophage phagocytosis (ADCP) data also showed some reactivity in this system, although the effect was relatively weaker than that of PD1 antibody, which was closely related to the high expression of CD38 antigen in cancer cells of patient 124#. Meanwhile weak expression of CD38 in patient 123# who did not respond to CD38-Ab monotherapy was only about 10.7% (FIG. 14). Therefore, the activity and function of NK cells and mononuclear macrophages (both of them were CD45+) was also retained during the collection of immune cells on day 0 in this system.

### 8. bioinformatics analysis

The bioinformatics fingerprints of overall tumor cells and immune cells of the patient cells in PVDF capsule tubes on day 0 was detected by molecular biological methods (RNA-seq and DNA-seq), so as to identify the biomarkers that may be reactive with immune drugs for the patient and reveal the molecular pathophysiological basis of disease occurrence and development.

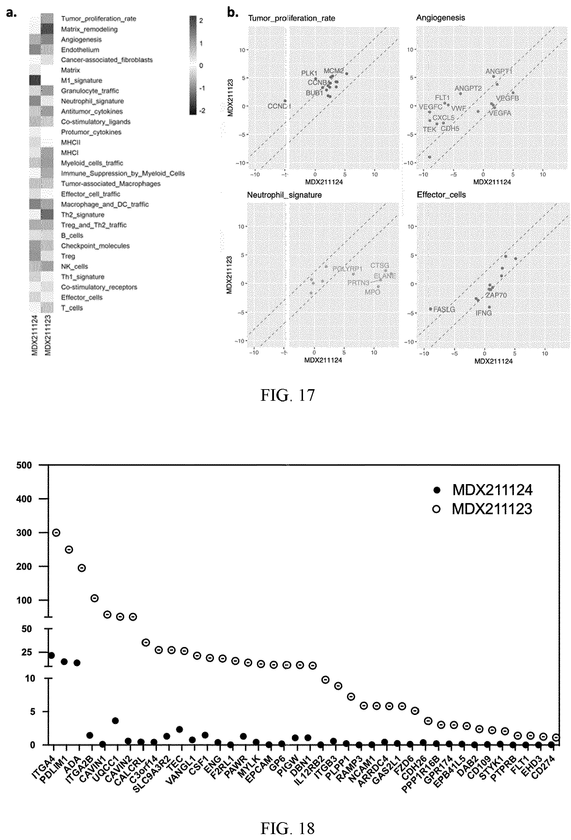

RNA-seq analysis of transcriptome differences between two patients:
(1) Analysis of differential gene signaling pathways: as shown in FIG. 17a and FIG. 17b, the immune non-responder patient #123 had high tumor proliferation activity (such as CCDN1, PLK1, MCM2), strong ability to remodel tumor microenvironment, high expression of neovascular associated markers (such as VEGFC, ANGPT2, FLT1, VWF), and low function of T cells, especially effector T cells. Immune responder patient #124 had low proliferation of tumor itself and low angiogenesis ability, but relatively powerful effector function of T cell (such as IFNγ producing) and neutrophils (such as ELANE, MPO, PRTN3).
(2) Potential key biomarkers identification for immunotherapy in AML patients (subsets of membrane protein): as shown in FIG. 18, the analysis of differential expression of membrane proteins indicated that compared with #124 immune responder patient, #123 immune non-responder patient significantly expressed higher levels of oncogenes or cancer susceptibility genes (ITGA4, ITGA 2B, ITGB3, PDLIM1, ADA, CAVIN1, CAVIN2, C3orf14, TEC, EPCAM, CD109, etc.), immune suppression related genes (CSF1, CD274/PDL1) and other membrane proteins that promote the occurrence and development of tumors, as well as showing expression of novel tumor targeting molecules that have not yet been reported, including SLC 9A3R2 (SLC 9A3 regulator 2), PIGW (phosphatidylinositol glycan anchor biosynthesis class W), CDH26 (cadherin 26) and PPP1R16B (protein phosphatase 1 regulatory subunit 16B). These four membrane molecules might not only be biomarkers of hematologic tumor, but also be the main potential biomarkers for the occurrence and development of broad spectrum of tumors (pan-cancers).

### DNA-seq analysis of genomic differences between two AML patients:

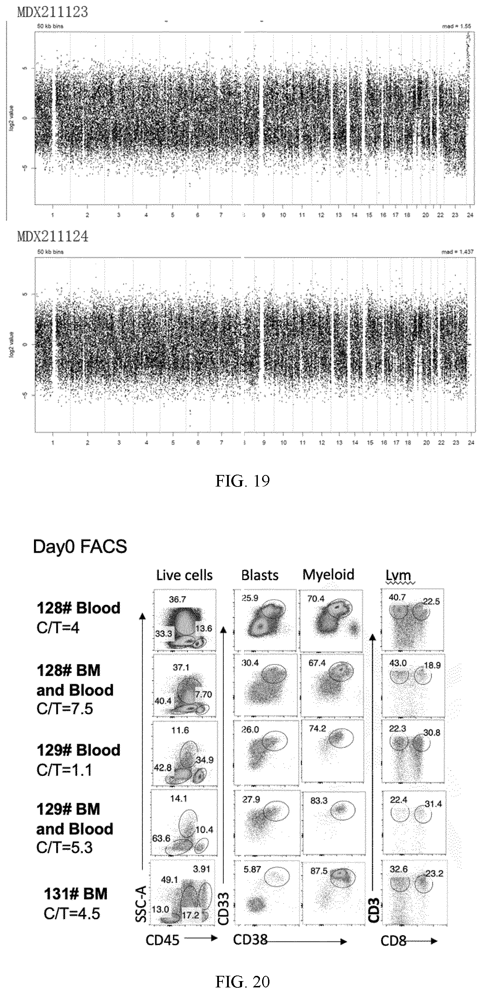

As shown in FIG. 19, the WES-CNV signal varied greatly, and there was no obvious deletion and amplification. Well-known biomarkers related to AML were not found, including fusion genes (ABL1-BCR, CRLF2, JAK2-BRAF, NOTCH1), mutant genes (FBXW7, JAK1, KRAS, NOTCH1, NPM1, FLT3, KIT, CEBPA), etc. That is to say, no key mutations of the reported genes were found in the two patients at the genome level.

### Example 6 Rapid functional screening and potential biomarker identification of immunotherapy for three patients with acute myeloid leukemia AML

### 1. Experimental animal preparation was the same as in Example 5.

### 2. Sample collection

Fresh peripheral blood samples and/or bone marrow from three AML patients (in the acute phase of AML patients) were collected, and placed in anticoagulant tubes, and transported to the central laboratory by cold chain (2-8°C) within a short period of time.

### 3. Sample processing and flow cytometry detection of the cells: the same as in Example 5.

### 4. Cell tubing

Similar to Example 4, the bone marrow samples and blood samples of some patients were mixed at 4:1 to 5:1 to ensure that the tumor tissue samples contained circulating peripheral blood cells, so as to maximally mimic function of human microenvironment. Quality control analysis was performed according to the data obtained by flow cytometry, so as to meet the requirement that the ratio of CD8+ T cells was at least more than 0.5% (the basic value of action of the immune drug), and the tumor cells (or non-CD 45high SSC-Alow cells) and the CD45high SSC-AlowCD3+ T lymphocytes were uniformly mixed according to a certain ratio (C/T (CD38+Cancer/CD3+T) =1.5:1 to 15:1), and then filled into PVDF tubes and sealed.

### 5. Inoculation and drug administration

The same as Example 4, the control groups and drug administration groups were randomly set (such as CD38 antibody, CD38 antibody combined with PD1 antibody, PD1 antibody combined with decitabine, PD1 antibody), and the drug administration was administered to the mice.

### 6. Data analysis

Flow cytometry sorting on day 0: as shown in FIG. 20, the blood C/T (CD38+Cancer/CD3+T) of patient 128# was 1:4, and the C/T of mixed bone marrow and blood sample was 1:7.5; the blood C/T of patient 129# was 1:1.1 (although more cancer blasts did not express CD38, that is to say, the C/T value of the patient was actually greater than 1:1.5), and the CT of mixed bone marrow and blood sample was 1:5.3. The C/T bone marrow sample of patient 131# was 1: 4.5. The myeloid cancer cells of these three patients all highly expressed CD38 molecules (about 70%-80%), and CD8+T precentage was also between 1% and 10%.

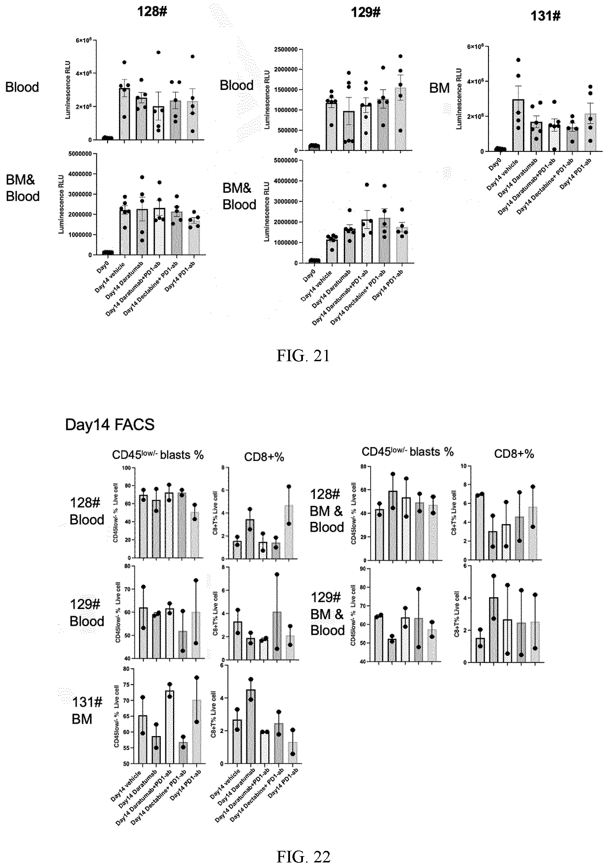

CTG cell viability on day 14: according to the viability value of the cells in the control group (as shown in FIG. 21), the proliferation viability of all the cells in the drug administration group was calculated, and then the efficacy of the drug was calculated.

Flow cytometry detection on day14: the relative ratio of tumor cells and immune cells in PVDF tubes was detected by flow cytometry. The differences between tumor cells and immune cells in the drug group and the control group were compared (as shown in FIG. 22). The results were analyzed by Flowjo software.

According to the results shown in FIG. 20, FIG. 21 and FIG. 22, although all the three patients highly expressed CD38 target protein, patients 128# and 129# hardly had a response effect to CD38 antibody. The data in FIG. 21 suggested that the total number of living cells did not decrease after drug treatment, and the data in FIG. 22 suggested that cancer blasts still occupied a dominant proportion in the mini-capsules. Meanwhile patient 131 # had a good response (FIG. 19). In addition, although three patients were also rich in TIL cells in bone marrow and/or blood (about 5%-17%), only patient 131 # had a better response to PD1 antibody, especially the combo-therapy of PD1 antibody in combination with decitabine (5aza).

The overall bioinformatics fingerprint of overall tumor cells and immune cells of the patients in PVDF capsule tubes on day 0 or day 14 was detected by molecular biological methods (RNA-seq and DNA-seq), so as to identify and evaluate the biomarkers of the patients sensitive and resistant to immune drugs.

### Example 7 Rapid functional screening of immunotherapy for a patient with acute myeloid leukemia AML

### 1. Experimental animals were the same as in Example 5.

### 2. Sample collection

Fresh peripheral blood and bone marrow samples from an AML patient (in the acute phase of AML) were collected, and placed in anticoagulant tubes, and transported to the central laboratory by cold chain (2-8°C) within a short period of time.

### 3. Cell processing, flow cytometry detection and data analysis were the same as in Example 5.

### 4. Cell tubing

Similar to Example 6, the bone marrow sample and blood sample of the patient were mixed at 4:1 to 5:1 to ensure the tumor tissue sample contained circulating peripheral blood cells, so as to maximally mimic the functional human microenvironment. Quality control analysis was performed according to the data of flow cytometry, it was found that the percentage of CD8 + T was 0.466% (close to the threshold value of 0.5%), and the ratio of tumor cells (CD38 + CD33 + tumor cells) and CD45high SSC-AlowCD3 + T was 1:8 (belonging to the range of C/T= 1.5:1 to 15:1). Downstream exploratory experiments were also performed, and the samples were filled into PVDF tubes and sealed.

### 5. Inoculation and administration

Similar to Example 4, the control groups and different administration groups were randomly setup (such as bcl2 inhibitor Venetoclax, CD38 antibody, CD38 antibody combined with Chidamide, CD38 antibody combined with 5AZA, CD38 antibody combined with Venetoclax, PD1 antibody, PD1 antibody combined with 5AZA, etc.), and the drug was administered.

### 6. Cell viability and phenotype detection were the same as in Example 5.

### 7. Data analysis

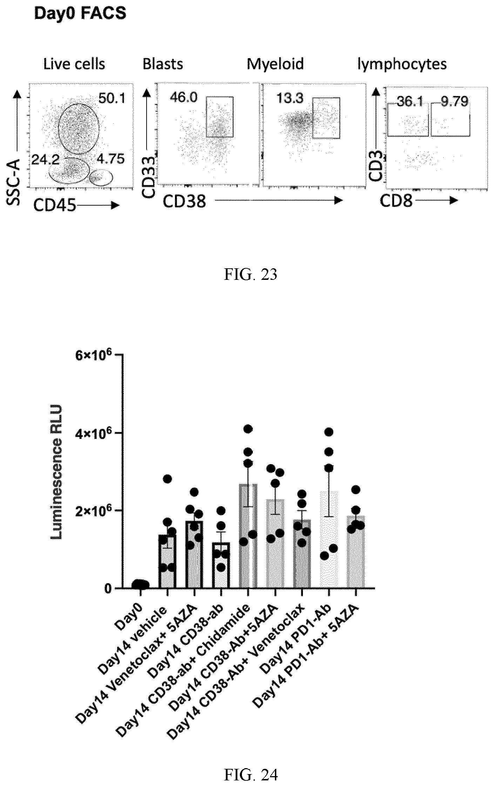

Flow cytometry (FACS) sorting on day 0: as shown in FIG. 23, C/T was 1:8 (although more tumor cells did not express CD38, that is to say, the C/T value of the patient was actually much larger than 8) for the mixed sample of bone marrow and blood of patient # 160, and the CD8 + T cell percentage was 0.466%.

CTG viability data on day 14: according to the viability value of the cells in the control group (as shown in FIG. 24), the proliferation viability of all the cells in the drug administration group was calculated, and then the efficacy of the drug was calculated.

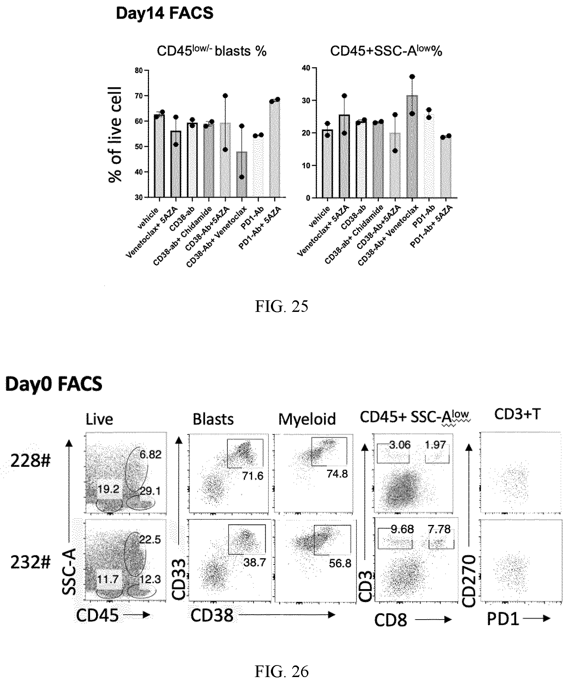

Flow cytometry detection on day14: the relative ratio of tumor cells and immune cells in PVDF tubes were detected by flow cytometry; the differences between tumor cells and immune cells in the drug group and the control group were compared (as shown in FIG. 25). The results were analyzed by Flowjo software.

According to the results of flow cytometry and CTG on day 14, CD45low/- tumor precursor/progenitor cells were predominant in mini-capsules, and the lymphocyte population accounted for about 20%. None of the tested drugs could significantly inhibit the cell activity of tumor tissue population. This experiment suggested that a high C/T ratio (>> 8) and a low CD8+% (<0.5%) may result in non-response to immunotherapy in the patient.

### Example 8 Rapid functional screening of immunotherapy for multiple (two) AML patients with acute myeloid leukemia wherein the same mouse was inoculated

### 1. Experimental animals were the same as in Example 4.

### 2. Sample collection

Fresh bone marrow samples from two AML patients (in the acute phase of AML) were collected, and placed in anticoagulant tubes, and transported to the central laboratory by cold chain (2-8°C) within a short period of time.

### 3. Cell processing, flow cytometry detection and data analysis, and cell tubing were the same as in Example 4.

### 4. Inoculation and drug administration

The capsules of bone marrow cells of two patients inoculated in the same mouse subcutaneously on both sides of the back of the mouse at 3 mini capsule tubes for each patient (two patients in one mouse). The mice were randomly grouped into control groups and the drug administration groups (such as CD38 antibody, PD1 antibody), and the mice in the administration group were administered the drug. At the same time, independent control and administration experiments (one patient in one mouse) were respectively carried out by a traditional method, and a part of mini capsule tubes on day10 were reserved and collected for FACS detection.

### 5. Cell viability and phenotype detection were the same as in Example 4.

### 6. Data analysis

Flow cytometry sorting on day 0: as shown in FIG. 26, for 228 # patient, bone marrow C/T (CD38 + Cancer/CD3 + T) was 1: 12, and the CD8+ cell percentage was 0.57 (higher C/T value and low CD8 + proportion, which suggested that the patient may not respond to T cell immunotherapy); for patient # 232, the bone marrow C/T ratio was 1:8.5, and the CD8+ cell percentage was 0.85%. The proportion of CD3 + T in both patients was low, and the expressions of PD1 and CD270 markers were also very weak, suggesting that the efficacy of PD1 antibody therapy for this patient may be not good.

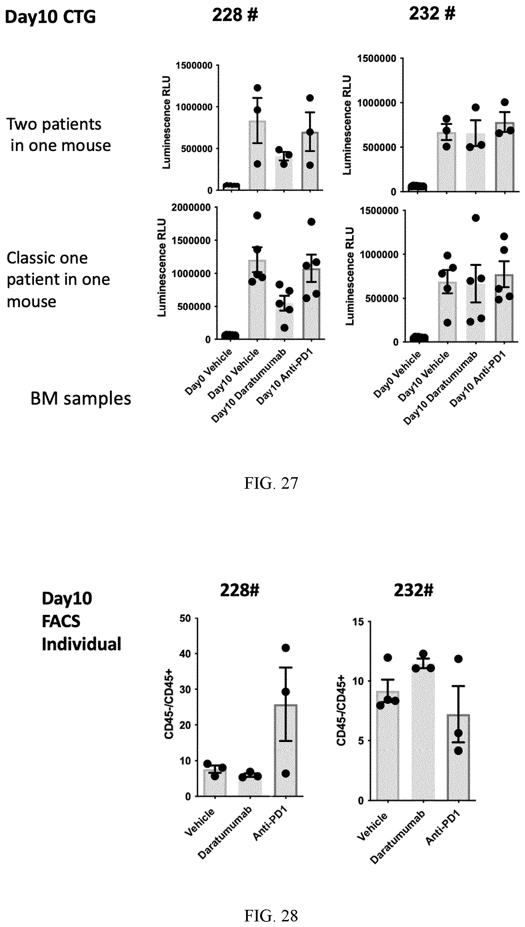

CTG cell viability on day 10: according to the viability value of the cells in the control group (as shown in FIG. 27), the proliferation viability of all the cells in the drug administration group was calculated, and then the efficacy of the drug was calculated.

Flow cytometry detection on day10: the relative ratio of tumor cells and immune cells in PVDF tubes was detected by flow cytometry; the differences between tumor cells and immune cells in the drug group and the control group were compared (as shown in FIG. 28). The results were analyzed by Flowjo software.

According to the results of CTG and FACS on day 10, CD45-tumor cells were predominant in the capsules of the two patients (as shown in FIG. 28). AML patient # 228 was sensitive to CD38 antibody therapy as the total cell viability was inhibited by about half (FIG. 27), and the ratio of CD45-tumor cells and CD45 + immune cells was also reduced compared with the control group (FIG. 28), while patient # 232 had no significant reactivity to CD38 antibody, but patient #232 also had higher expression of CD38 antigen (FIG. 26 and FIG. 27). The reason for this difference in response did not exclude that it was caused by the difference in the density of CD38 tumor antigen of patients and the functional difference of bone marrow myeloid immune cells such as NK, monocyte macrophage and neutrophil such as ADCC and ADCP. Nevertheless, it was consistent with the earlier prediction that both patients would not respond to PD1 antibodies, possibly due to the fact that very few PD1 + CD3 + T cells in tumor microenvironment were present in the patient sample (FIG. 26 and FIG. 27). In addition, the data of drug susceptibility detection of "two patients in one mouse" model was also consistent with the data of our traditional model "one patient in one mouse" (FIG. 27). Therefore, for the rapid screening of efficacy of immune drugs in vivo, the same mouse can be used to load capsules of multiple patients for testing in order to save costs and resources, which can be distinguished and traced according to different inoculation sites (the present experiment), or size of different capsules, or even different color coding of the capsules.

The important significance of the immunological drug functional screening method in vivo is summarized:
(1) The data in Example 1, Example 2 and Example 3 suggested that the method was good for finding that some ovarian cancer patients rich in TIL with weak initial PD1/PDL1 expression still had a good response to PD1 antibody. Significant or differential changes of CD270 expression in tumor-infiltrating lymphocytes were also likely to be a biomarker for PD1 antibody therapy (no studies have been reported at present). Even when the PD1 expression was abundant in tumor tissue, if the ratio and abundance of tumor cells and immune cells were unbalanced, the response of immunotherapy can also be affected, and the combination of immunotherapy and targeting therapy may still be an effective way to overcome the non-response of mono-therapy (this argument was also supported by Example 4).
(2) The data in Example 5, Example 6 and Example 7 suggested that the method can well screen and distinguish differential responses to CD38 or PD1 antibodies in vivo
   between CD38+/high and (or) TIL enriched patients (such as AML) with blood tumor. In addition to the discovery of well-known cancer-driver genes or cancer susceptibility genes (these reported genes accounted for about more than 90% of the overall differential genes of membrane molecules that we analysed, indirectly proving the reliability of the model), and new potential diagnostic and therapeutic targets have been found: SLC9A3R2 (SLC9A3 regulator 2), PIGW (phosphatidylinositol glycan anchor biosynthesis class W), CDH26 (cadherin 26) and PPP1R16B (protein phosphatase 1 regulatory subunit 16B). Therefore, this rapid-functional experiment-mediated omics analysis was conducive to the discovery and research of new targets.
(3) Example 8 suggested that the same mouse can be loaded with tumor capsules from multiple patients with the same disease for rapid efficacy screening of immunological drugs in vivo, and the results were proved to be consistent with the drug sensitivity experiment of traditional immunodeficiency mice loaded with tumor capsules from one patient, which was beneficial for cost saving and efficiency improvement.

## Claims

1. A polyvinylidene fluoride (PVDF) capsule tube encapsulating cells, wherein the cells comprise tumor cells and immune cells in a single cell suspension, and further wherein the tumor cells and immune cells are derived from fresh tumor tissues or body fluids of clinical patients;
wherein the ratio of tumor cells to immune cells (CD3+T cell) ranges from 1.5: 1 to 15: 1, and/or,
wherein the percentage of CD8+ T cells in total live cells is above 0.5%;
wherein the immune cells are autologous immune cells enriched by sorting into a single cell suspension, and the tumor cells are those that flow through when sorting the single cell suspension, wherein the single cell suspension is derived from tumor-infiltrating lymphocytes in solid tumor tissue of a patient, and/or the peripheral blood mononuclear cells in peripheral blood of a patient.

2. The polyvinylidene fluoride (PVDF) capsule tube encapsulating cells of claim 1, wherein the tumor tissues comprise clinically surgically resected tumor tissues or biopsied tumor tissues.

3. The polyvinylidene fluoride (PVDF) capsule tube encapsulating cells of claim 1, wherein the body fluids comprise one or more of blood, bone marrow, pleural fluid and ascites and cerebrospinal fluid.

4. The polyvinylidene fluoride (PVDF) capsule tube encapsulating cells of any one of claims 1 to 3, wherein the average protein permeability of the polyvinylidene fluoride (PVDF) capsule tube is around 300-1,000 kDa, or 500-700 kDa.

5. The polyvinylidene fluoride (PVDF) capsule tube encapsulating cells of any one of claims 1 to 4, wherein the polyvinylidene fluoride (PVDF) capsule tube is pre-processed by activating, flushing with ultrapure water and autoclaving before use.

6. The polyvinylidene fluoride (PVDF) capsule tube encapsulating cells of claim 5, wherein activating is accomplished by rinsing the polyvinylidene fluoride (PVDF) capsule tube in dehydrated alcohol.

7. The polyvinylidene fluoride (PVDF) capsule tube encapsulating cells of any one of claims 1 to 6, wherein the sorting method used in said sorting comprises sorting immune cells by adherence to magnetic beads conjugated with anti-human CD45.

8. The polyvinylidene fluoride (PVDF) capsule tube encapsulating cells of any one of claims 1 to 6, wherein the sorting method used in said sorting comprises sorting tumor cells by deleting immune cells and fibroblast cells using magnetic beads conjugated with anti-human CD45 and anti-human fibroblast cell markers.

9. The polyvinylidene fluoride (PVDF) capsule tube encapsulating cells of any one of claims 1-8, wherein the ratio of tumor cells to immune cells (CD3+T cell) ranges is from 1.5: 1 to 9: 1.

10. The polyvinylidene fluoride (PVDF) capsule tube encapsulating cells of any one of claims 1-9, wherein the percentage of CD8+ T cells in total live cells is from 0.5% to 15%, and, the viability of the immune cells is more than 20%.

11. An animal model comprising at least one polyvinylidene fluoride (PVDF) capsule tube of any one of claims 1-10, wherein the polyvinylidene fluoride (PVDF) capsule tube is implanted subcutaneously or orthotopically; the animal is a mouse; preferably, the mouse is an immunodeficient mouse selected from the group consisting of a BALB/c Nude mouse, a NCG mouse, a NSG mouse, and a NOD-scid mouse.

12. The animal model of claim 11, wherein the number of the polyvinylidene fluoride (PVDF) capsule tube is 1-8.

13. A method for rapid screening the efficacy of immunoregulating drugs, comprising the following steps:
(1) administering a drug to be tested to the animal model of claim 11 or claim 12, wherein the drug is an immunoregulating drug, wherein the drug is in a solid, semi-solid or liquid form; preferably, the drug is administered by intravenous injection, oral gavage, intraperitoneal injection, or subcutaneous injection;
(2) detecting the total cell viability of tumor cells and immune cells or immune cell viability in the polyvinylidene fluoride (PVDF) capsule tube and the relative ratio and cell phenotype of tumor cells and immune cells; preferably, the detecting method of the total cell viability or immune cell viability is detected by the chemiluminescence method, and the detecting method of relative ratio and cell phenotype by flow cytometry;
(3) detecting molecular biological information of tumor cells and immune cells in the polyvinylidene fluoride (PVDF) capsule tube; preferably, the detection method of biological information is a DNA or RNA sequence; and wherein, steps (2) and (3) can be completed in any order; more preferably, the flow cytometry measures cellular expression of 7AAD, CD45, CD3, CD8, CD270, CD33, CD38, CD20, PDL1, PD1, CD68 and CD25.

14. Use of the capsule tube of any one of claims 1 to 10 for the preparation of an animal model for rapid screening of the efficacy of an immunoregulating drug; the animal is an immunodeficient mouse; preferably, capsules of more than one patient are simultaneously implanted and each capsule contain cells that can be derived from different patients.

## Patentansprüche

1. Polyvinylidenfluorid-(PVDF)-Kapselrohr, das Zellen verkapselt, wobei die Zellen Tumorzellen und Immunzellen in einer Einzelzellsuspension umfassen, und wobei die Tumorzellen und Immunzellen weiter von frischen Tumorgeweben oder Körperflüssigkeiten klinischer Patienten gewonnen werden;
wobei das Verhältnis von Tumorzellen zu Immunzellen (CD3+T-Zelle) im Bereich von 1,5:1 bis 15:1 liegt, und/oder,
wobei der Prozentsatz von CD8+T-Zellen in den insgesamt lebenden Zellen über 0,5 % liegt;
wobei die Immunzellen autologe Immunzellen sind, angereichert durch Sortieren in einer Einzelzellsuspension, und die Tumorzellen jene sind, die bei Sortieren der Einzelzellsuspension durchfließen, wobei die Einzelzellsuspension aus tumorinfiltrierenden Lymphozyten in solidem Tumorgewebe eines Patienten und/oder den mononukleären peripheren Blutzellen im peripheren Blut eines Patienten gewonnen wird.

2. Polyvinylidenfluorid-(PVDF)-Kapselrohr, das Zellen nach Anspruch 1 verkapselt, wobei die Tumorgewebe klinisch chirurgisch resezierte Tumorgewebe oder durch Biopsie erhaltene Tumorgewebe umfassen.

3. Polyvinylidenfluorid-(PVDF)-Kapselrohr, das Zellen nach Anspruch 1 verkapselt, wobei die Körperflüssigkeiten eines oder mehrere umfassen von Blut, Knochenmark, Pleuraflüssigkeit und Ascites und cerebrospinaler Flüssigkeit.

4. Polyvinylidenfluorid-(PVDF)-Kapselrohr, das Zellen nach einem der Ansprüche 1 bis 3 verkapselt, wobei die durchschnittliche Proteinpermeabilität des Polyvinylidenfluorid-(PVDF)-Kapselrohrs etwa 300-1.000 kDa oder 500-700 kDa beträgt.

5. Polyvinylidenfluorid-(PVDF)-Kapselrohr, das Zellen nach einem der Ansprüche 1 bis 4 verkapselt, wobei das Polyvinylidenfluorid-(PVDF)-Kapselrohr vorverarbeitet ist durch Aktivierung, Spülung mit ultrapurem Wasser und Autoklavbehandlung vor Verwendung.

6. Polyvinylidenfluorid-(PVDF)-Kapselrohr, das Zellen nach Anspruch 5 verkapselt, wobei Aktivierung durch Spülen des Polyvinylidenfluorid-(PVDF)-Kapselrohrs in dehydriertem Alkohol erreicht wird.

7. Polyvinylidenfluorid-(PVDF)-Kapselrohr, das Zellen nach einem der Ansprüche 1 bis 6 verkapselt, wobei das bei dem Sortieren verwendete Sortierverfahren Sortieren von Immunzellen durch Adhärenz an mit anti-humanem CD45 konjugierten magnetischen Kügelchen umfasst.

8. Polyvinylidenfluorid-(PVDF)-Kapselrohr, das Zellen nach einem der Ansprüche 1 bis 6 verkapselt, wobei das bei dem Sortieren verwendete Sortierverfahren Sortieren von Tumorzellen durch Eliminieren von Immunzellen und Fibroblasten-Zellen unter Verwendung von mit anti-humanem CD45 konjugierten magnetischen Kügelchen und antihumanen Fibroblasten-Zellmarkern umfasst.

9. Polyvinylidenfluorid-(PVDF)-Kapselrohr, das Zellen nach einem der Ansprüche 1-8 verkapselt, wobei das Verhältnis von Tumorzellen zu Immunzellen (CD3+T-Zelle) im Bereich von 1,5:1 bis 9:1 liegt.

10. Polyvinylidenfluorid-(PVDF)-Kapselrohr, das Zellen nach einem der Ansprüche 1-9 verkapselt, wobei der Prozentsatz von CD8+T-Zellen in den insgesamt lebenden Zellen von 0,5 % bis 15 % beträgt und die Lebensfähigkeit der Immunzellen mehr als 20 % beträgt.

11. Tiermodell, umfassend mindestens ein Polyvinylidenfluorid-(PVDF)-Kapselrohr nach einem der Ansprüche 1-10, wobei das Polyvinylidenfluorid-(PVDF)-Kapselrohr subkutan oder orthotop implantiert wird; wobei das Tier eine Maus ist; wobei die Maus vorzugsweise eine immungeschwächte Maus ist, ausgewählt aus der Gruppe bestehend aus einer BALB/c-Nacktmaus, einer NCG-Maus, einer NSG-Maus und einer NOD-scid Maus.

12. Tiermodell nach Anspruch 11, wobei die Anzahl des Polyvinylidenfluorid-(PVDF)-Kapselrohrs 1-8 beträgt.

13. Verfahren zum schnellen Screening der Wirksamkeit von immunregulierenden Medikamenten, das die folgenden Schritte umfasst:
(1) Verabreichen eines zu testenden Medikaments an das Tiermodell nach Anspruch 11 oder Anspruch 12, wobei das Medikament ein immunregulierendes Medikament ist, wobei das Medikament in einer festen, halbfesten oder flüssigen Form vorliegt; wobei das Medikament vorzugsweise durch intravenöse Injektion, Schlundsonde, intraperitoneale Injektion oder subkutane Injektion verabreicht wird;
(2) Detektieren der Gesamt-Zelllebensfähigkeit von Tumorzellen und Immunzellen oder der Immunzellen-Lebensfähigkeit in dem Polyvinylidenfluorid-(PVDF)-Kapselrohr und des relativen Verhältnisses und des Zellphänotys von Tumorzellen und Immunzellen; wobei das Detektionsverfahren der Gesamt-Zelllebensfähigkeit oder Immunzellen-Lebensfähigkeit durch das Chemilumineszenz-Verfahren detektiert wird und das Detektionsverfahren des relativen Verhältnisses und des Zellphänotyps durch Durchflusszytometrie detektiert wird;
(3) Detektieren von molekularbiologischen Informationen von Tumorzellen und Immunzellen in dem Polyvinylidenfluorid-(PVDF)-Kapselrohr; wobei das Detektionsverfahren von biologischen Informationen eine DNA- oder RNA-Sequenz ist; und wobei die Schritte (2) und (3) in beliebiger Reihenfolge abgeschlossen werden können; wobei die Durchflusszytometrie vorzugsweise zelluläre Expression von 7AAD, CD45, CD3, CD8, CD270, CD33, CD38, CD20, PDL1, PD1, CD68 und CD25 misst.

14. Verwendung des Kapselrohrs nach einem der Ansprüche 1 bis 10 zur Herstellung eines Tiermodells zum schnellen Screening der Wirksamkeit eines immunregulierenden Medikaments; wobei das Tier eine immungeschwächte Maus ist; wobei vorzugsweise die Kapseln von mehr als einem Patienten gleichzeitig implantiert werden und jede Kapsel Zellen enthält, die von verschiedenen Patienten gewonnen werden können.

## Revendications

1. Tube-capsule en polyfluorure de vinylidène (PVDF) encapsulant des cellules, dans lequel les cellules comprennent des cellules tumorales et des cellules immunitaires dans une suspension monocellulaire, et en outre dans lequel les cellules tumorales et les cellules immunitaires sont dérivées de tissus tumoraux frais ou de fluides corporels de patients cliniques ;
dans lequel le rapport de cellules tumorales à cellules immunitaires (cellule T CD3+) varie de 1,5: 1 à 15: 1, et/ou,
dans lequel le pourcentage de cellules T CD8+ dans le total des cellules vivantes est supérieur à 0,5 % ;
dans lequel les cellules immunitaires sont des cellules immunitaires autologues enrichies par tri en une suspension monocellulaire, et les cellules tumorales sont celles qui s'écoulent lors du tri de la suspension monocellulaire, dans lequel la suspension monocellulaire est dérivée de lymphocytes infiltrant la tumeur dans le tissu tumoral solide d'un patient, et/ou des cellules mononucléées du sang périphérique dans le sang périphérique d'un patient.

2. Tube-capsule en polyfluorure de vinylidène (PVDF) encapsulant des cellules selon la revendication 1, dans lequel les tissus tumoraux comprennent des tissus tumoraux réséqués chirurgicalement sur le plan clinique ou des tissus tumoraux biopsiés.

3. Tube-capsule en polyfluorure de vinylidène (PVDF) encapsulant des cellules selon la revendication 1, dans lequel les fluides corporels comprennent un ou plusieurs éléments parmi le sang, la moelle osseuse, le liquide pleural et l'ascite et le liquide céphalorachidien.

4. Tube-capsule en polyfluorure de vinylidène (PVDF) encapsulant des cellules selon l'une quelconque des revendications 1 à 3, dans lequel la perméabilité moyenne aux protéines du tube-capsule en polyfluorure de vinylidène (PVDF) est d'environ 300-1 000 kDa, ou 500-700 kDa.

5. Tube-capsule en polyfluorure de vinylidène (PVDF) encapsulant des cellules selon l'une quelconque des revendications 1 à 4, dans lequel le tube-capsule en polyfluorure de vinylidène (PVDF) est prétraité par activation, rinçage à l'eau ultrapure et autoclavage avant utilisation.

6. Tube-capsule en polyfluorure de vinylidène (PVDF) encapsulant des cellules selon la revendication 5, dans lequel l'activation est réalisée par rinçage du tube-capsule en polyfluorure de vinylidène (PVDF) dans de l'alcool déshydraté.

7. Tube-capsule en polyfluorure de vinylidène (PVDF) encapsulant des cellules selon l'une quelconque des revendications 1 à 6, dans lequel le procédé de tri utilisé dans ledit tri comprend un tri de cellules immunitaires par adhérence à des billes magnétiques conjuguées à de l'anti-CD45 humain.

8. Tube-capsule en polyfluorure de vinylidène (PVDF) encapsulant des cellules selon l'une quelconque des revendications 1 à 6, dans lequel le procédé de tri utilisé dans ledit tri comprend un tri de cellules tumorales par suppression de cellules immunitaires et de cellules fibroblastiques en utilisant des billes magnétiques conjuguées à de l'anti-CD45 humain et à des marqueurs de cellules fibroblastiques anti-humains.

9. Tube-capsule en polyfluorure de vinylidène (PVDF) encapsulant des cellules selon l'une quelconque des revendications 1 à 8, dans lequel le rapport de cellules tumorales à cellules immunitaires (cellule T CD3+) varie de 1,5: 1 à 9: 1.

10. Tube-capsule en polyfluorure de vinylidène (PVDF) encapsulant des cellules selon l'une quelconque des revendications 1 à 9, dans lequel le pourcentage de cellules T CD8+ dans le total des cellules vivantes est de 0,5 % à 15 %, et, la viabilité des cellules immunitaires est supérieure à 20 %.

11. Modèle animal comprenant au moins un tube-capsule en polyfluorure de vinylidène (PVDF) selon l'une quelconque des revendications 1 à 10, dans lequel le tube-capsule en polyfluorure de vinylidène (PVDF) est implanté par voie sous-cutanée ou de manière orthotopique ; l'animal est une souris ; de préférence, la souris est une souris immunodéficiente sélectionnée parmi le groupe consistant en une souris BALB/c Nude, une souris NCG, une souris NSG, et une souris NOD-scid.

12. Modèle animal selon la revendication 11, dans lequel le nombre du tube-capsule en polyfluorure de vinylidène (PVDF) est 1 à 8.

13. Procédé de criblage rapide de l'efficacité de médicaments immunorégulateurs, comprenant les étapes suivantes :
(1) administration d'un médicament devant être testé au modèle animal selon la revendication 11 ou la revendication 12, dans lequel le médicament est un médicament immunorégulateur, dans lequel le médicament est sous une forme solide, semi-solide ou liquide ; de préférence, le médicament est administré par injection intraveineuse, gavage oral, injection intrapéritonéale, ou injection sous-cutanée ;
(2) détection de la viabilité cellulaire totale de cellules tumorales et de cellules immunitaires ou de la viabilité de cellules immunitaires dans le tube-capsule en polyfluorure de vinylidène (PVDF) et du rapport relatif et du phénotype cellulaire de cellules tumorales et de cellules immunitaires ; de préférence, le procédé de détection de la viabilité cellulaire totale ou de la viabilité de cellules immunitaires est détecté par la méthode par chimiluminescence, et le procédé de détection du rapport relatif et du phénotype cellulaire par cytométrie en flux ;
(3) détection d'informations biologiques moléculaires de cellules tumorales et de cellules immunitaires dans le tube-capsule en polyfluorure de vinylidène (PVDF) ; de préférence, le procédé de détection d'informations biologiques est une séquence d'ADN ou d'ARN ; et dans lequel, les étapes (2) et (3) peuvent être effectuées dans n'importe quel ordre ; de manière davantage préférée, la cytométrie en flux mesure l'expression cellulaire de 7-AAD, CD45, CD3, CD8, CD270, CD33, CD38, CD20, PDL1, PD1, CD68 et CD25.

14. Utilisation du tube-capsule selon l'une quelconque des revendications 1 à 10 pour la préparation d'un modèle animal pour le criblage rapide de l'efficacité d'un médicament immunorégulateur ; l'animal est une souris immunodéficiente ; de préférence, des capsules de plus d'un patient sont implantées simultanément et chaque capsule contient des cellules qui peuvent être dérivées de différents patients.
